# EUROPÄISCHE PATENTSCHRIFT

(11) **EP 1 385 949 B1**
(45) Veröffentlichungstag und Bekanntmachung des Hinweises auf die Patenterteilung: **23.08.2006**
(21) Anmeldenummer: 02719800.1
(22) Anmeldetag: 11.02.2002
(51) Int. Cl.: C12N 15/10, C12Q 1/68

(54) **VERFAHREN ZUR HERSTELLUNG VON GENBIBLIOTHEKEN**
METHOD FOR PRODUCING GENE LIBRARIES
PROCEDE DE FABRICATION DE BIBLIOTHEQUES GENETIQUES

(30) Priorität: 28.02.2001 DE 10109517
(43) Veröffentlichungstag der Anmeldung: 04.02.2004
(73) Patentinhaber: Sieber, Volker, 85395 Wolfersdorf (DE)
(72) Erfinder: Sieber, Volker, 85395 Wolfersdorf (DE)
(74) Vertreter: Strehl Schübel-Hopf & Partner
(86) Internationale Anmeldenummer: PCT/EP2002/001418
(87) Internationale Veröffentlichungsnummer: WO 2002/068634

(56) Entgegenhaltungen:
- MURAKAMI HIROSHI ET AL: "Random insertion and deletion of arbitrary number of bases for codon-based random mutation of DNAs." NATURE BIOTECHNOLOGY, Bd. 20, Nr. 1, Januar 2002 (2002-01), Seiten 76-81, XP002241084 January, 2002 ISSN: 1087-0156
- EISINGER DOMINIC P ET AL: "Long-inverse PCR to generate regional peptide libraries by codon mutagenesis." BIOTECHNIQUES, Bd. 22, Nr. 2, 1997, Seite 250, 252, 254 XP002241075 ISSN: 0736-6205
- HAYES F ET AL: "Pentapeptide scanning mutagenesis: encouraging old proteins to execute unusual tricks." TRENDS IN MICROBIOLOGY. ENGLAND DEC 2000, Bd. 8, Nr. 12, Dezember 2000 (2000-12), Seiten 571-577, XP002241076 ISSN: 0966-842X
- KEGLER-EBO D ET AL: "Codon cassette mutagenesis: a general method to insert or replace individual codons by using universal mutagenic cassettes" NUCLEIC ACIDS RESEARCH, OXFORD UNIVERSITY PRESS, SURREY, GB, Bd. 22, Nr. 9, 1994, Seiten 1593-1599, XP002103322 ISSN: 0305-1048
- CHEN W-J ET AL: "A MODIFIED KANAMYCIN-RESISTANCE CASSETTE TO FACILITATE TWO-CODON INSERTION MUTAGENESIS" GENE (AMSTERDAM), Bd. 111, Nr. 1, 1992, Seiten 143-144, XP002241077 ISSN: 0378-1119
- DATABASE WPI Section Ch, Week 200309 Derwent Publications Ltd., London, GB; Class B04, AN 2003-096535 XP002241237 & JP 2002 253255 A (KAGAKU GIJUTSU SHINKO JIGYODAN), 10. September 2002 (2002-09-10)

### Sequenzprotokoll

<110> Sieber, Volker
<120> Verfahren zur Herstellung von Genbibliotheken
<130> PCA-53830
<150> DE 10109517
   <151> 2001-02-28
<160> 4
<170> Patent In Ver. 2.0
<210> 1
   <211> 48
   <212> DNA
   <213> künstliche Sequenz
<220>
   <223> Beschreibung der künstlichen Sequenz: primer for amplifying chloramphenicol acetyltransferase
<220>
   <221> misc_feature
   <222> (1,2)
   <223> n=a or g or c or t
<220>
   <221> misc_feature
   <222> (3)
   <223> s=g or c
<400> 1
   nnsgggcctg ggtctcctcc tggcgagaaa aaaatcactg gatatacc 48
<210> 2
   <211> 24
   <212> DNA
   <213> künstliche Sequenz
<220>
   <223> Beschreibung der künstlichen Sequenz: primer for amplifying chloramphenicol acetyltransferase
<400>
   ggcgtagctc ctcgcgttta aggg 24
<210> 3
   <211> 21
   <212> DNA
   <213> künstliche Sequenz
<220>
   <223> Beschreibung der künstlichen Sequenz: primer for amplifying gfp
<400> 3
   ccatgattac gccaagcttg c 21
<210> 4
   <211> 22
   <212> DNA
   <213> künstliche Sequenz
<220>
   <223> Beschreibung der künstlichen Sequenz: primer for amplifying chloramphenicol acetyltransferase
<400> 4
   gtgcttattt ttctttacgg tc 22

## Beschreibung

### Gebiet der Erfindung

Die hier dargestellte Erfindung bezieht sich auf eine Methode zur Herstellung von Sequenzvariation in DNA. Sie kann speziell zur Herstellung von Bibliotheken aus Genen, die für Proteine kodieren und die sich untereinander durch Mutationen (Austausch) gesamter Codons auszeichnen, verwendet werden. Durch Verfahren der Selektion oder des *Screening* können aus diesen Bibliotheken Genvarianten isoliert werden, die für Proteine mit besonderen Eigenschaften, wie z.B. eine veränderte Aktivität oder erhöhte Stabilität kodieren.

### Hintergrund der Erfindung

In der biotechnologischen und chemischen Industrie, sowie in der medizinischen Diagnostik und Therapie werden zunehmend Enzyme verwendet'. Sie verbessern herkömmliche oder ermöglichen völlig neue Prozesse und Anwendungen. Diese Möglichkeiten wecken einen großen Bedarf an Enzymen mit neuen oder verbesserten Eigenschaften. Die Optimierung der Enzyme durch rationale oder rechnergestützte Methoden ist bisher weitestgehend erfolglos². Ein dagegen recht erfolgreiches Verfahren, zum Optimieren von Enzymeigenschaften, ist die gerichtete Evolution von Proteinen³. Dabei wird zunächst durch Variation einer oder mehrerer DNA-Sequenzen des entsprechenden Enzyms eine Genbibliothek hergestellt, aus der anschließend durch Selektion oder durch Durchmusterung (*Screening*) diejenigen Genvarianten isoliert werden, die für Enzymvarianten kodieren, die die gewünschten, optimierten Eigenschaften besitzen. Der Vorteil dieser Vorgehensweise liegt darin, dass keine Informationen über Strukturen der Enzyme, ihre Dynamik oder ihre. Wechselwirkungen mit verschiedenen Substraten notwendig sind. Methoden der gerichteten Evolution werden daher zunehmend von, sehr vielen Biotechnologiefirmen weltweit akzeptiert und angewendet ^{1, 4}.
Der Erfolg einer gerichteten Evolution, hängt vor allem von der Qualität der Bibliotheken und von der Effektivität der Selektions-und Durclimusterungsstrategien ab. Die Qualität einer Bibliothek ist durch die darin verfügbare Sequenzvariation bestimmt und hängt weitestgehend von dem Verfahren ab, mit dem die Sequenzvariation gebildet wird_ Heutzutage werden hauptsächlich zwei Wege der Sequenzvarüerung angewandt: *in-vitro* DNA-Rekombination⁵⁻⁷ und Zufallsmutagenese⁸⁻¹⁰.

Am Anfang der *in-vitro* DNA-Rekombination steht ein kleines Repertoire an DNA-Sequenzen, die untereinander sehr ähnlich, aber nicht identisch sind. Die Unterschiede (Mutationen) zwischen den Sequenzen können aus der natürlichen Evolution stammen (family shuffling)¹¹ oder durch die Kombination von Zufallsmutagenese und Selektion eingeführt worden sein⁵. Sie stellen daher eine Vorauswahl dar und daher verbessern sie zum großen Teil die Enzymeigenschaften oder sind zumindest neutral.

Bei der *in-vitro* DNA-Rekombination wird ein Repertoire an DNA-Sequenzen hergestellt, dessen Varianten neue Kombinationen der im ursprünglichen Repertoire vorhandenen Mutationen enthalten. Der Vorteil dieser Methode ist, dass sie eine größere Zahl an Positionen in einem Gen gleichzeitig variieren kann und dabei meist günstige Mutationen miteinander verbindet. Der Nachteil hingegen ist, dass sich ein Verfahren der Rekombination immer nur auf Positionen beschränkt, die innerhalb des ursprünglichen Repertoires schon Variationen gezeigt haben und außerdem nur auf die an diesen Positionen vorhandenen Mutationen.

Bei der Zufallsmutagenese dagegen werden völlig neue Mutation eingebaut Ihr Vorteil ist, dass an allen Positionen eines Gens alle möglichen Variationen eingebaut werden können, sie sich also nicht auf die Vereinigung schon vorhandener Mutation beschränkt.
Zwei Techniken der Einführung von zufälligen Mutationen in ein Gen werden heutzutage fast ausschließlich angewandt: die fehlerhafte PCR⁸ und die ortsspezifische (Kassetten-) Mutagenese mittels degenerierter Oligonukleotide⁹.

### Fehlerhafte PCR

Die im Moment bei weitem dominierende Methode der ortsunspezifischen Zufallsmutagenese ist die fehlerhafte PCR. Dabei wird das zu mutierende Gen einer PCR unter Verwendung einer thermostabilen Polymerase unterzogen (meist Taq-Polymerase), die abhängig von den Bedingungen der Reaktion einzelne Basen falsch in die synthetisierten Gene einbaut¹². Eine häufige Variante dieser Methode beinhaltet die Verwendung von Mangan(II)-Ionen⁸ oder von NukJeotidanaloga¹³ im PCR Ansatz. Diese Methode ist sehr preiswert und einfach durchzufnhren. Dennoch hat sie die folgenden, einschneidenden Nachteile:
1.) Durch die Beschaffenheit des genetischen Codes - drei Nukleotide kodieren eine Aminosäure, jede Aminosäure wird durch bis zu 6 verschiedene Codons kodiert - lassen sich aus einzelnen Nukleotidaustauschen durchschnittlich nur 6 neue Aminosäuren erreichen. Viele Aminosäureaustausche sind nur durch zwei und manche gar nur durch drei Nukleotidaustausche möglich. Zum Beispiel kann ein Codon für Isoleucin (ATT, ATA oder ATC) nur durch zwei Nukleotidaustausche aus dem Codon CAA für Glutamin und nur durch drei Austausche aus dem Codon CAG für Glutamin gebildet werden. Beispiele aus der Anwendung von Mutagenese zur Verbesserung von Enzymen belegen, dass Varianten mit den geforderten Eigenschaften oft tatsächlich nur durch Triplettaustausche, also Austausche aller drei Nukleotide eines Codons erhalten werden können^{14, 15}.
   Ein Repertoire an Varianten eines Proteins, bei dem an allen Positionen alle möglichen Aminosäuren einzeln ausgetauscht vorkommen, muss, wenn es durch einzelne Nukleotidaustausche (z.B. fehlerhafte PCR) erhalten wurde, sehr groß sein. Ein durchschnittliches Protein von 300 Aminosäuren Länge hat genau 300 x 19 = 5700 verschiedene Varianten, die sich in einer Aminosäure von ihm unterscheiden. Ein Repertoire an Varianten dieses Proteins, was durch durchschnittlich drei Nukleotidaustausche erhalten wurde, hat dagegen (900 x 3)^3 = 2x10¹⁰ unterschiedliche Varianten. Eine derartige Zahl an Varianten kann nur von den wenigsten Selektionsmethoden behandelt werden¹⁶. Die Methode der fehlerhaften PCR zur Mutagenese kann daher nicht eingesetzt werden, um vollständige bzw. hochqualitative Repertoires eines Proteins zu erhalten.
2.) Bei der fehlerhaften PCR werden nicht alle Nukleotide gleichermaßen ausgetauscht. Es kommt in unterschiedlicher Frequenz zu Transversionen und Transitionen¹⁷, manche Mutaüonen treten so viel häufiger auf, als andere. Hierdurch wird eine weitere Verkleinerung der effektiven Größe eines durch fehlerhafte PCR gewonnen Repertoires eingeführt.
3.) Durch die hohe Redundanz der Codons - mehrere Codons kodieren für die selbe Aminosäuren kommt es beim Austausch einzelner Nukleotide durchschnittlich zu 23 % zu synonymen Mutationen, bei denen das mutierte Codon für die selbe Aminosäure kodiert, wie das ursprüngliche. Zwar können diese Mutationen zu günstigen Veränderungen bei der Expression von Protein führen¹⁸, wichtige intrinsische Eigenschaften eines Proteins, wie z.B. seine enzymatische Aktivität oder Stabilität bleiben dabei aber unbeeinflusst und somit wird die effektive Größe eines Repertoires herabgesetzt.
4.) Im Durchschnitt werden durch 4 % aller Nukleotidaustausche Stopcodons eingeführt. Wenn die Mutageneserate hoch gewählt wird, um eine ausreichend hohe Zahl an Aminosäureaustauschen zu erhalten, so können dadurch eine große Zahl an Stopcodons eingeführt werden, was zu abgeknrzten Genprodukten führt. Bei einer durchschnittlichen Mutationsrate von nur 3 Austauschen pro Gen, die theoretisch notwendig ist, um wenigstens an einer Position alle möglichen Aminosäuren einzuwechseln, sind schon über 10 % des Repertoires durch vorzeitige Stopcodons verkürzt [1-(1-0.04)^3] = 0. 115.
5.) Der Einbau von Mutationen ist ein weitgehend statistischer Prozess; bei dem die Anzahl der Mutationen in den einzelnen Varianten einer Poissonverteilung folgt. Dadurch können abhängig von der durchschnittlichen Mutationsraté erhebliche Teile des Repertoires ohne Mutation sein, während andere über sehr viele Mutationen verfügen. Eine weitere Verkleinerung der effektiven Größe des Repertoires ist die Folge.
6.) Der Einbau unnatürlicher Aminosäuren bei der Biosynthese von Proteinen kann erreicht werden, wenn modifizierte Komponenten des Proteintranslationsystems und neue Codons mit vier, statt mit drei Basen verwendet werden¹⁹. Um Varianten von Proteinen herzustellen, die derartige unnatürliche Aminosäuren an zufälligen Positionen enhalten, müssen gesamte Codons, das heisst drei Basen gegen vier Basen ausgetauscht werden. Durch fehlerhafte PCR ist dies effektiv nicht möglich.

### Ortsgerichtete Zufallsmutagenese

Eine Alternative zur fehlerhaften PCR ist, mittels degenerierter Oligonukleotide und PCR an verschiedenen, genau definierten Positionen in einem Gen gezielt mehrere Nukleotide gleichzeitig auszutauschen. Ein Beispiel für eine derartige Methode geben Eisinger et al.³⁵ an. Sie beschreiben, wie Codons von einem in einem Plasmid befindlichen Gen mittels zweier Primer, von denen der eine degenerierte Oligonukleotide enthält, durch eine PCR, gefolgt von einer Ligationsreaktion variiert werden können. Diese und ähnliche Methoden^{20-22,34} bleiben aber zwangsläufig darauf beschränkt, dass mit jeder Reaktion nur ein spezifisches Codon variiert werden kann, für das mindestens ein Primer entwickelt werden muss. Für jede neue zu variierende Position muss entsprechend mindestens ein neuer Primer in einer neuen Reaktion verwendet werden. Werden alle drei Nukleotide eines Codons beliebig variiert, kann an diesen Positionen im Repertoire jede natürliche Aminosäure kodiert sein²⁰. Allerdings ist das so erhaltene Repertoire sehr ungleichmäßig, Arginin und Serin kommen z.B. 6 mal so häufig vor, wie Methionin oder Tryptophan. Außerdem kann der Einbau von Stopcodons problematisch werden.
Durch Kombinationen bzw. Mischungen bestimmter Nukleotide an verschiedenen Positionen läßt sich der Grad der Degeneriertheit der Oligonukleotide aber so einstellen, dass sich diese Probleme teilweise umgehen lassen, indem z. B. DNA-Sequenzrepertoires erhalten werden, die nur einen spezifischen Teil aller Aminosäuren kodieren oder die alle Aminosäuren mit gleicher Wahrscheinlichkeit enthalten^{21,22}
Eine auf dem Papier bessere Alternative zur Nukleotidmischung stellt der Einbau von Nukleotidtripletts, also gesamten Codons statt einzelnen Nukleotiden bei der chemischen Synthese von DNA-Strängen dar²³⁻²⁷. Leider unterscheiden sich die verschiedenen Trinukleotide in der Effizienz der chemischen Kopplung, was wiederum zu ungleichen Repertoires führt²⁷. Aber auch wenn diese chemischen Probleme gelöst werden können, bleibt diese Methode zwangsläufig auf ortsspezifische Zufallsmutagenese beschränkt.

Die hier dargestellten Einschränkungen der derzeit angewandten Methoden der Zufallsmutagenese zeigen deutlich, wie sehr vorteilhaft es wäre, wenn es eine Methode gäbe, die es ermöglichte,
a) ganze Codons (unabhängig von der Zahl der Basen pro Codon) statt einzelner Nukleotide in DNA-Strängen zufälligen auszutauschen und
b) eine definierten Zahl an Mutationen (Codonaustausche) pro DNA-Strang einzubauen.
Die hier dargelegte Erfindung beschreibt genau eine solche Methode. Mit ihr hergestellte Repertoires an Genvarianten besitzen eine höhere Qualität als solche, die durch herkömmliche Methoden der fehlerhaften PCR hergestellt werden können. Sie enthalten eine größere Zahl an verschiedenen Varianten bei gleicher Anzahl an Mitgliedern. Gekoppelt mit einer Selektion oder einem *Screening* lassen sich aus diesen Repertoires effizienter Varianten isolieren, die für Proteine mit neuen und gewünschten Eigenschaften kodieren. Diese Proteine können dann z.B. als Enzyme in der biotechnologischen Industrie, in der medizinischen Diagnostik oder Therapie eingesetzt werden.

Weitere Vorteile und Anwendungsmöglichkeiten werden offenbar werden durch die unten folgende Beschreibung der Erfindung und die dazugehörigen Abbildungen.

### Beschreibung der Abbildungen

### Abb. 1:

(A) Schematische Darstellung des Einbaus eines Donor-DNA-Strangs (dunkler Kasten) an zufälligen Positionen eines Gens (heller Kasten), das sich in einer zirkulären Form (z.B. Plasmid) eingebunden befindet. Der Donor-Strang kann das Gen eines Reporterproteins tragen.
(B) Der Donor-Strang wird so entfernt, dass das ursprüngliche Gen wieder entsteht, dabei aber ein Codon (hier als Beispiel CAG bzw. GTG) durch ein anderes Codon (hier als Beispiel ATT bzw. TAC) ersetzt wird.
Die Zahlen entsprechen einer beispielhaften Nummerierung der Aminosäuren.

### Abb. 2:

Detaillierte schematische Darstellung der einzelnen wesentlichen Schritte zur Einführung von Codon-Mutationen in ein Gen. Für die genauere Erläuterung siehe die Beschreibung der Erfindung im Text. Die gestrichelte Linie verdeutlicht die Zirkularität des Gens, sie steht zum Beispiel für ein Expressionsplasmid. Die Zahlen entsprechen einer beispielhaften Nummerierung der Aminosäuren. Die Pfeile über den Genen kennzeichnen Restriktionsschnittstellen (nicht Erkennungsstellen) der Restriktionsenzyme R1, R2, R3 und R4.

### Abb. 3:

Detaillierter Verlauf der Mutagenese am Beispiel des Austauschs des Codons 86 von GFP (ÄGT, nt 256-258) gegen TGG (Aminosäurenmutation: S86W). (i) ... Einführung eines Doppelstrangbruchs, (ii) ... Einbau des Donorstrangs, (iii) ... Restriktion mit *Bse*RI, (iv)... Glättung der DNA-Enden, (v) ... Religation.
Oberhalb der DNA Sequenzen sind Nukleotide nummeriert, unterhalb sind Codons (Aminosäuren) nummeriert. Die Erkennungsstellen für *Bse*RI sind fett dargestellt, die Schnittstellen durch gestrichelte Linien markiert.

### Abb. 4:

Agarosegelelektrophorese von PCR-Produkten zur Analyse der Einbauposition der Donor-DNA in das GP Gen. Die Leiter gibt die Größe der Fragmente in bp an.

### Definitionen

Vor der detaillierten Beschreibung der Erfindung werden zunächst einige Begriffe definiert:

*DNA* (deoxyribonucleic acid), *Polynukleotid, Nukleotidsequenz* oder *Oligonukleotid* bezeichnet jede Kette oder Sequenz der chemischen Bausteine Adenin (A), Cytosin (C), Guanin (G) und Thymin (T) (Nukleotidbasen). Nukleotide und Oligonukleotide sind *degeneriert,* wenn sie an einer oder mehreren Positionen mehr als eine Nukleotidbase enthalten können. DNA kann einen Strang von Nukleotidbasen enthalten (*Einzelstrang*) oder zwei gegenseitig komplementäre Stränge (*Doppelstrang*), die eine Doppelhelixstruktur bilden. Bei einem *Einzelstrangbruch* wird einer der Stränge eines Doppelstrangs gespalten, wobei der Doppelstrang erhalten bleibt. Bei einem *Doppelstrangbruch* werden beide Stränge des Doppelstrangs gespalten und es entstehen zwei neue DNA Enden. *Glatte DNA Enden* bedeuten, dass an den Enden von DNA Doppelsträngen beide Stränge gleich lang sind, bei *überhängenden DNA Enden* ist dagegen einer der Stränge länger als der andere. Bei einer *blunt-end-Ligation* werden zwei Doppelstränge mit glatten DNA Enden kovalent miteinander verbunden.
Die Begriffe *Bibliothek, Repertoire* oder *Ensemble,* wie sie hier verwendet werden, ersetzen sich gegenseitig und bezeichnen eine Sammlung von Polynukleotiden oder Polypeptiden. Eine *Gen-* oder *DNA-Bibliothek* oder ein *Repertoire an Gen oder DNA-Sequenzen* ist eine Sammlung von Polynukleotiden oder DNA-Sequenzen, die von einem oder mehreren Polynukleotiden oder DNA-Sequenzen abgeleitet sind und mit diesen größtenteils identisch sind. Jedes einzelne Polynukleotid oder jede einzelne DNA-Sequenz der Bibliothek wird als *Mitglied* der Bibliothek bezeichnet. Mehrere Mitglieder der Bibliothek, die untereinander identisch sind, entsprechen einer *Genvariante* oder einer *Variante* der Bibliothek. Die *effektive Größe* einer Bibliothek oder eines Repertoires ist ein Maß für die Zahl der unterschiedlichen Varianten in dieser Bibliothek. Eine große Bibliothek hat sehr viele Mitglieder, hätte aber bei geringer effektiver Größe nur wenige verschiedene Varianten.
Enzyme bzw. Begriffe der Molekularbiologie wie *Restriktionsenzym, Restriktionsenzym des Typs IIs, DNase I, Nuklease, Exonuklease, DNA-Ligase, DNA-Polymerase, Transposase, Transposon, Vektor* und *Plasmid* sind in ihrer Funktion so definiert, wie in einschlägigen Werken der Molekularbiologie beschrieben^{28,29}.

### Genaue Beschreibung der Erfindung

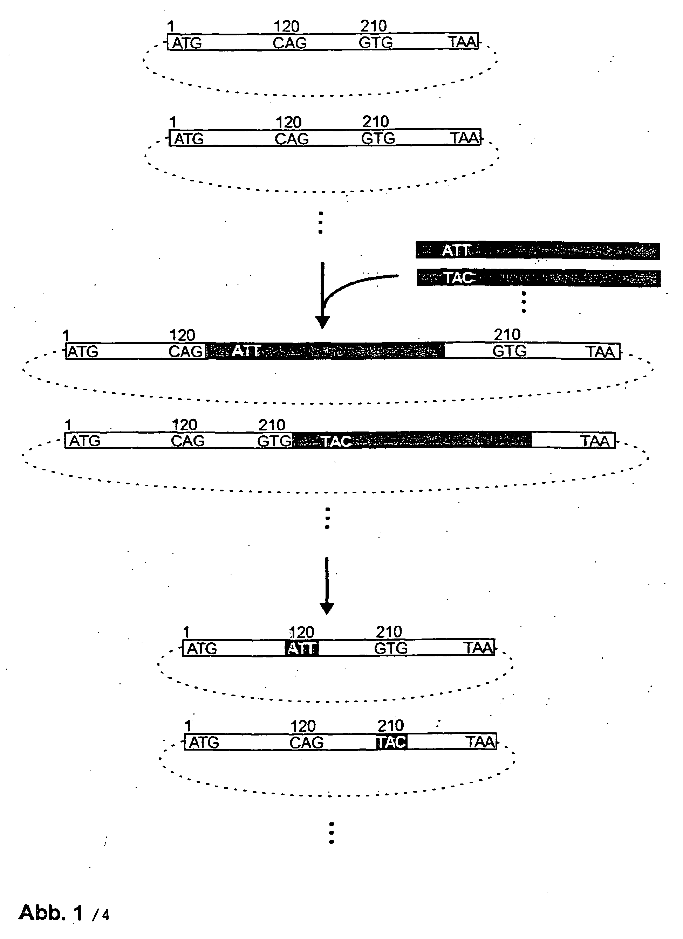

Die Erfindung setzt sich zusammen aus (siehe auch Abb. 1):
A.) Der Einführung eines DNA Abschnitts in ein Gen an verschiedenen, zufälligen Positionen.
B.) Dem gezielten Herausschneiden dieses DNA-Abschnitts und einer definierten Anzahl an nebeneinanderliegenden Nukleotiden des besagten Gens unter Hinterlassung einer definierten Anzahl von Nukleotiden aus dem eingeführten DNA-Abschnitt an deren Stelle.

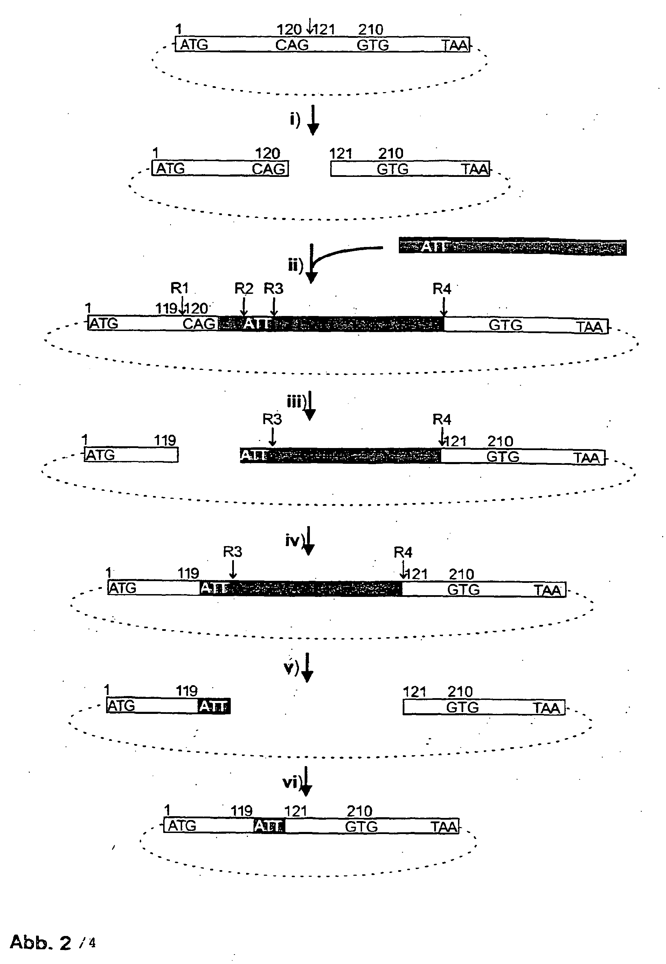

Im Detail zeichnet sich die Erfindung durch folgende Arbeitsschritte aus, siehe dazu auch Abb. 2:
1.) In Moleküle eines Gens bzw. einer DNA Sequenz; bevorzugt eingebunden in einem Vektor oder in einer anderweitig zirkularen Form, wird durch molekularbiologische Methoden an zufälligen Positionen pro Molekül genau je ein Doppelstrangbruch verursacht (Abb.2, i). Das kann z.B. erreicht werden, indem die DNA behandelt wird mit a) einem Enzym, das sequenzunspezifisch einen Einzelstrangbruch verursacht (z.B. DNase I) und einer einzelstrangspezifischen Nuklease (z.B. S 1 Nuklease), b) einem Enzym, das sequenzspezifisch einen Einzelstrangbruch verursacht, einer 5'-3' Exonuklease, DNA-Polymerase und einzelstrangspezifischen Nuklease (z.B. S1 Nuklease), c) einem Enzym das sequenzunspezifisch Doppelstrangbrüche verursacht (z.B. modifizierte Varianten von Restriktionsenzymen, die ihre Sequenzspezifität verloren haben) oder d) einem Transposon und einer Transposase, die keine Sequenzspezifität besitzt. Bevorzugt entsteht dadurch ein Ensemble an Genvarianten, bei denen sich die Doppelstrangbrüche an unterschiedlichen Positionen befinden und (außer im Fall d) glatte DNA Enden verursachen. Im Fall d) erfolgt der Doppelstrangbruch unter gleichzeitigem Einbau eines DNA Strangs (Transposon) und eventuell einer Verdoppelung mehrerer Nukleotide des Gens.
2.) In die Genvarianten dieses Ensembles wird durch blunt-end-Ligation ein DNA Strang (Donor-Strang) eingeführt (Abb. 2, ii). Im Falle der Verwendung einer Transposase (ld) ist der Donor-Strang als Transposon bereits durch den vorangegangen Schritt in das Gen eingebaut. Bevorzugt kodiert der Donor-Strang für einen genetisch selektierbaren Marker, z.B. für eine Antibiotikaresistenz. Weiterhin bevorzugt ist, dass dieser Marker abhängig von der Einordnung des eingesetzten Donor-Strangs in den Leserahmen des Gens exprimierbar ist. Der Donor-Strang enthält bevorzugt Erkennungsstellen für Restriktionsenzyme des Typs IIs. Die so erhaltenen DNA Konstrukte können entweder vollständig oder nur Teile davon mit PCR^{30,31} amplifiziert werden und/oder nach Transformation von Mikroorganismen in diesen amplifiziert und aus diesen isoliert werden. Bevorzugt erfolgt das Wachstum der Mikroorganismen in Medium mit einem Antibiofikum wogegen die Mikroorganismen nur dank eines Genprodukts resistent sind, das auf dem Donor-Strang kodiert ist.
3.) Durch Restriktion mit besagten Restriktionsenzymen des Typs 11s werden die Donor-Stränge zum größten Teil wieder aus den amplifizierten Genvarianten entfernt und durch die Behandlung mit einer DNA Polymerase oder einzelstrangspezifischen Nuklease werden die DNA Enden glatt gemacht (Abb. 2, iii). Die Positionen der Restriktionsschnittstellen des Typs 11s sind bevorzugt so gewählt, dass dabei außerdem eine definierte Zahl n Nukleotide aus dem ursprünglichen Gen mit entfernt wird und dafür eine definierte Zahl m Nukleotide aus dem ursprünglichen Donor-Strang in den Genabschnitten verbleiben. Diese verbleibenden m Nukleotide sind bevorzugt degeneriert, das heißt in den Genvarianten des Ensembles verbleiben verschiedene Nukleotidzusammensetzungen. Bevorzugt werden genau 3 Nukleotide ersetzt (n=3, m=3).
   Ist die Variabilität der Nukleotidzusammensetzung in der näheren Umgebung (heißt 10 bis 40 Basenpaare) der Enden des Donor-Strangs eingeschränkt, z.B. durch konservierte Sequenzen eines Transposons, so enthält dieser bevorzugt mehrere Erkennungsstellen für Restriktionsenzyme des Typs IIs. Diese sind bevorzugt so gelegen, dass durch mehrere Zyklen von a) Restriktion mit einem oder zweien dieser Enzyme, b) wenn notwendig Behandlung zu glatten DNA Enden und c) anschließender Fusion der DNA Enden durch intramolekulare Ligation, der Donor-Strang stückweise aus dem Gen herausgeschnitten wird, bis er vollständig mit Ausnahme einer definierten Zahl m Nukleotiden entfernt worden ist unter gleichzeitiger Entfernung einer definierten Zahl n Nukleotiden aus dem ursprünglichen Gen (Abb. 2, iv und v). Diese verbleibenden m Nukleotide sind bevorzugt degeneriert, es werden bevorzugt genau 3 Nukleotide ersetzt (n=3, m=3).
4.) Durch intramolekulare blunt-end-Ligation werden die DNA-Enden der Varianten des Ensembles wieder geschlossen und vollständige, kontinuierliche Gene wieder gewonnen (Abb. 2, vi). Die Gene können einer weiteren Runde des Einbaus von Mutationen unterzogen werden oder alternativ zur Expression der durch sie kodierten Proteinvarianten verwendet werden, entweder *in vivo* nach Transformation eines Expressionsorganismus oder *in vitro* mit Hilfe eines *in vitro -* Translationssystems.

Die hier offengelegte Methode ist die bisher einzige Mutagenesemethode, die den zufälligen Austausch von mehreren nebeneinanderliegenden Nukleotide in einem Gen erlaubt Es sind bisher verschiedene molekularbiologische Methoden veröffentlicht worden, die auf zufälligen Doppelstrangbrüchen in DNA-Strängen und auch zum Teil auf den Einbau von DNA-Sequenzen, auch von Transposons, an zufälligen Positionen in diese DNA-Stränge basieren. Diese Methoden beschränken sich aber auf Versuche, neue Termini für Proteine zu finden³², zufällige Abschnitte aus Proteinen zu löschen oder definierte zusätzliche Sequenzen in Proteine einzufügen³³. Ein solche Methode ist beispielsweise die Pentapeptid Scanning Mutagenese, wie sie Hayes et al. beschreiben³⁶. Durch zufallsbedingte Insertion eines Transposons in ein Gen und nachfolgender gerichteter Entfernung eines größeren Teils des Transposons werden in dieser Methode in unterschiedlichen Positionen eines Genes 15 Nukleotide eingeführt. Damit werden Proteinbanken erhalten, deren Varianten je nach Leserahmen, in dem die Nukleotide in das Gen eingebaut wurden, an zufälligen Positionen 5 spezifische Aminosäuren enthalten. Eine Methode, bei der durch den Einbau eines DNA-Abschnitts (eine sog. Kassette) in ein Gen und die anschließende Entfernung des größten Teils dieser Kassette auch Nukleotide des Gens gezielt ersetzt werden können, beschreiben Kegler-Ebo et al.³⁷. Bei der von ihnen entwickelten Methode wird das Gen an einer spezifischen Stelle aufgeschnitten und die Kassette durch blunt-end Ligation eingeführt. Je nach Design der Kassette können bei ihrer anschließenden Entfernung zusätzliche Codons in das Gen eingebaut, Codons des Gens gelöscht oder auch ersetzt werden. Diese Methode ist eine Weiterentwicklung der Methode von Chen et al.³⁸, die ähnlich funktioniert, sich allerdings auf den Einbau von Nukleotiden (2 Codons) beschränkt und nicht erlaubt Nukleotide auszutauschen. Gemeinsam ist beiden Methoden, dass der Ort der Variation (Einbau, Deletion oder Ersatz) genau festgelegt ist und zudem für jede Reaktion spezifisch vorbereitet werden muss. Sie sind nicht dazu verwendet worden, und allein auch nicht dazu verwendbar, Nukleotide an zufälligen Positionen in DNA-Sequenzen so auszutauschen, dass einzelne Aminosäuren in den davon kodierten Proteinen ausgetauscht werden und so Repertoires von Genen entstehen, deren Produkte sich untereinander nur in der Art einer definierten Zahl ihrer Aminosäuren unterscheiden.

### Was wird vorausgesehen:

Es wird vorausgesehen, dass der Anteil jeder theoretisch möglichen Mischung an Nukleotiden in den degenerierten Bereichen der Donor-DNA genau eingestellt werden kann, z.B. dahingehend, dass in einem Bereich von 3 degenerierten Nukleotiden genau jede Aminosäure durch genau ein Codon vertreten ist. Es wird vorausgesehen, dass es mit der hier dargestellten Methode möglich ist, Mutationen nicht nur in die gesamte Länge eines Gens sondern auch in beschränkte Bereiche von Genen einzuführen. Dazu können z. B. diese Bereiche nach Einführung der Donor-Stränge mit flankierenden Primern durch PCR amplifiziert werden, die Amplifikate über GenSOEing³⁴ in das vollständige Gen wieder eingeführt und das modifizierte Gen dem beschriebenen Protokoll weiter unterzogen werden. Es wird auch vorausgésehen, dass bei der stufenweisen Entfernung der Donor-Stränge die notwendigen Restriktionsschnittstellen des Typs IIs erst nach einzelnen Stufen kreiert werden z.B. nach Restriktion und Religation. Es wird vorausgesehen, dass in Donor-Strängen, die durch Wirkung einer Transposase als Transposon in Gene eingebaut werden, Mutationen in den für die Transponierung notwendigen Transposase-Erkennungsstellen eingeführt werden können, die in diesen Bereichen neue Erkennungsstellen für Restriktionsenzyme des Typs IIs erzeugen. Es wird vorausgesehen, dass weitere Techniken möglich sind, Doppelstrangbrüche in DNA einzuführen, als die in der Beschreibung der Erfindung beispielhaft angegebenen. Es wird vorausgesehen, dass durch Immobilisierungstechniken Gene mit eingebauten Donor-Strängen von Genen, die keine Donor-Strängen enthalten oder Donor-Strängen, die in Gene eingebaut sind, von Donor-Strängen, die nicht in Gene eingebaut sind, physikalisch abgetrennt werden können.

### Ausführungsbeispiel

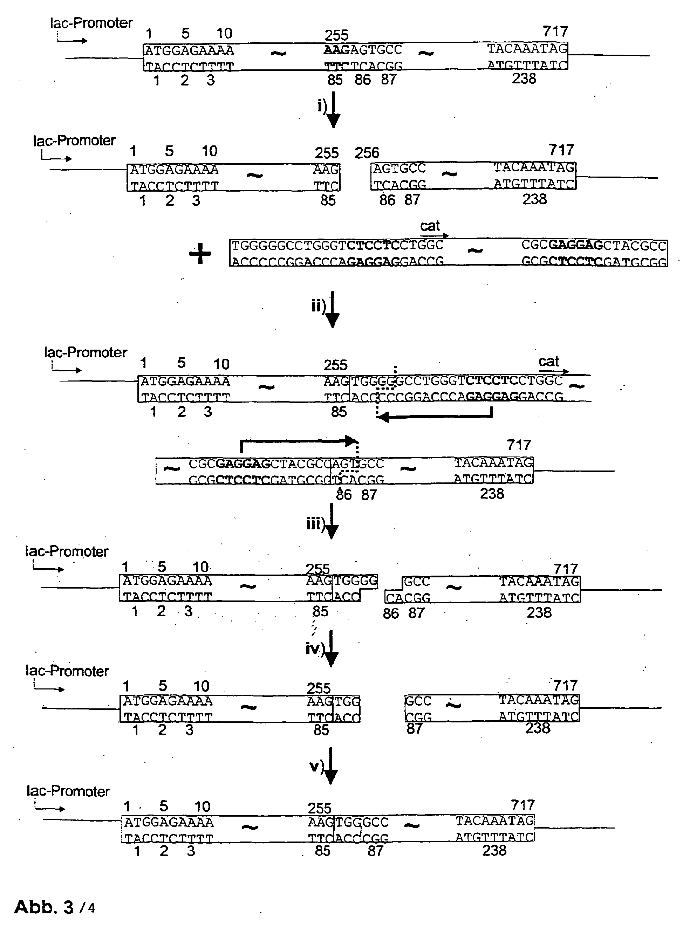

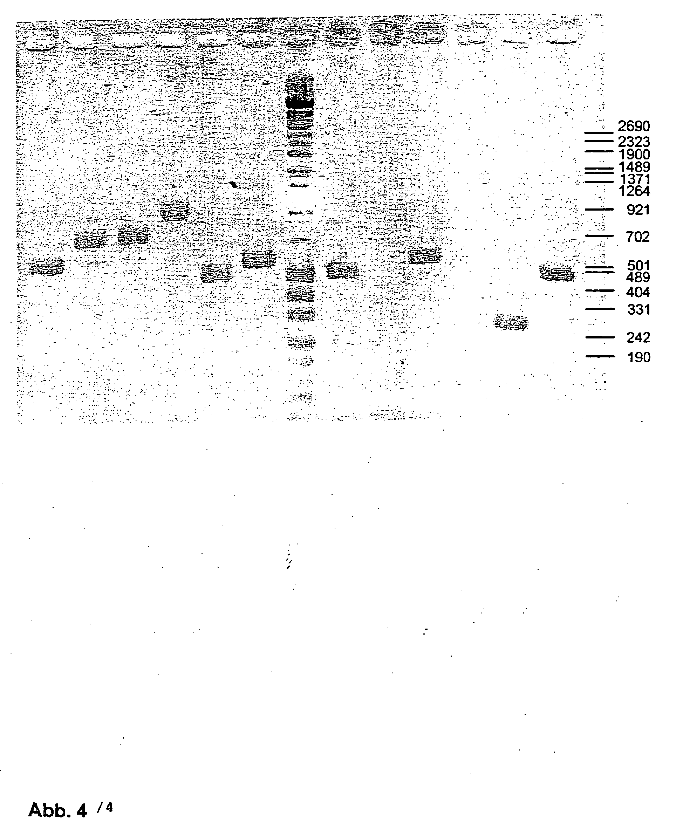

### Beispiel 1- Einbau von Codonmutationen in das Gen des Grün Fluoreszierenden Proteins (GFP), (siehe dazu auch Abb. 3 und 4)

### 1.) Einführung von zufälligen Doppelstrangbrüchen in pGFP

Das Plasmid pGFP (Clontech, Palo Alto, USA) enthält das GFP-Gen unter der Kontrolle des Lac-Promoters. ZurAmplifikation in *E.Coli* enthält es das Gen für Ampicillin: *E.coli* XL1-Blue Zellen wurden mit pGFP transformiert und aus 200 ml einer Kultur der transformierten Zellen wurden 300 µg pGFP präpariert (Maxikit, Quiagen, Hilden).
In 200 µl 33 mM Tris/HCl, pH 7,5, 10 mM MgCl₂ und 50µg/ml BSA wurden 40 µg pGFP 5 min bei 28 °C inkubiert mit 0,01 mu DNase I (Roche Diagnostik, Penzberg). Die Reaktion wurde durch Zugabe von 20 mM EDTA (Endkonz.) und Kühlung auf Eis abgestoppt Die Analyse der Reaktion durch Agarosegelelektrophorese zeigte, dass ca. 40 % von pGFP in die offen-zirkuläre Form umgewandelt worden war. Mit präparativer Agarosegelelektrophorese wurde diese Form isoliert. In 100 µl 7.4x S1 Puffer (MBI Fermentas, St. Leon Roth) wurden 5 µg der offen-zirkulären Form mit 100 u S1 Nuklease (1 µl, MBI Fermentas, St Leon Roth) für 2 h bei 16 °C inkubiert und die Reaktion anschließend mit 10 µl S1-Stop Lösung abgestoppt Die Analyse der DNA Probe per Agarosegelelektrophorese ergab, dass ca. 50 % der offen-zirkulären DNA linearisiert wurde. Diese linearisierte DNA wurde mit präparativer Agarosegelelektrophorese isoliert.

### 2.) Präparation des einzuführenden DNA Strangs (Donor-Strang)

Das Gen von Chloramphenicolacetyltransferase (CAT) wurde in einer PCR mit den Primern NNS GGG CCT GGG TCT CCT CCT GGC GAG AAA AAA ATC ACT GGA TAT ACC (SEQ. ID NO:1) und GGC GTA GCT CCT CGC GTT TAA GGG (SEQ. ID NO: 2) und dem Plasmid pACYC184 (NEB, Beverly, MA, USA) als Matrize amplifiziert. Die PCR wurde nach Standard angesetzt (NEB, Beverly, MA, USA), es wurden 30 Zyklen durchgeführt mit einer Annealingtemperatur von 55°C und einer Annealingzeit von 45 sec. Es wurde Vent-Polymerase verwendet (NEB, Beverly, MA, USA). Das PCR Produkt wurde mit EtOH gefällt und in einem kleinem Volumen TE zu einer Konzentration von 150 ng/µl aufgenommen.

### 3.) Einführung des Donor-Strangs in das Plasmid und Transformation.

In 50 µl Ligasepuffer (Gibco BRL, Eggenstein) (Endvolumen) werden 10 µl linearisiertes Plasmid (ca. 300 ng, siehe 1.) und 14 µl PCR Produkt (ca. 2 µg, siehe 2.) mit 5 u T4-DNA Ligase (Gibco BRL, Eggenstein) für 20 h bei 16°C inkubiert. Anschließend wurde der Ligationsansatz durch Mikrodialyse entsalzt und zur Transformation von XL1-Blue Zellen durch Elektroporation eingesetzt. Die transformierten Zellen wurden auf dYT-Agar mit 100 µg/ml Ampicillin und 8 µg/ml Chloramphenicol und 1 mM IPTG ausplattiert. Wachstum von transformierten Bakterien konnte weitestgehend nur erfolgen, wo Zellen mit Plasmiden transformiert sind, bei denen die PCR Fragmente in einen Bereich unter der Kontrolle des lac-Promotors eingebaut werden und der Einbau zusätzlich auch noch im richtigen Leserahmen nach einem Startcodon erfolgt. Es wurden ca. 10000 Transformanten erhalten.

### 4.) Bibliotheksanalyse

Von 95 Kolonien wurde mittels Kolonie-PCR und den Primern CCA TGA TTA CGC CAA GCT TGC **(SEQ. ID NO: 3)** (bindet am 5'-Ende des GFP-Gens und GTG CTT ATT TTT CTT TAC GGT C **(SEQ. ID NO: 4)** (bindet innerhalb des CAT-Gens) untersucht, ob ein Einbau der PCR-Produkte in die Plasmide innerhalb oder außerhalb des Gens von GFP erfolgte und, wenn ja, ob bei Einbau in das GFP-Gen dieser in der richtigen Leserichtung und mit einer zufälligen Verteilung erfolgte. Von ca. 80 % der Transformanten ließ sich ein Fragment der Länge zwischen ca. 270 und ca. 1000 bp amplifizieren (siehe z.B. Abb. 4a). Das heißt, dass bei diesen Varianten der Einbau des PCR-Produkts innerhalb der Gensequenz von GFP erfolgte und dabei das Gen für Chloramphenicolacetyltransferase in der selben Leserichtung liegt, wie das Gen für GFP.

### 5.) Donor-Entfemung

Alle Kolonien der transformierten Bakterien wurden von den Agarplatten entfernt und vereint und aus ihnen plasmidale DNA präpariert (Mini kit, Quiagen, Hilden). 2 µg der Plasmid DNA, welche ein Repertoire an pGFP mit zufällig eingesetztem PCR Produkt darstellt, wurden präparativ mit *Bse*RI verdaut (NEB, Beverly, MA, USA). Dieses Restriktionsenzym vom Typ IIs schneidet außerhalb seiner Erkennungsstelle CTCCTC (Abb. 3, üi). Die Produkte der Restriktionsreaktion wurden mit Klenowfragment behandelt, anschließend durch Agarosegetelektrophorese aufgetrennt und die DNA Bande bei ca. 3,4 kb aus der Agarose präpariert (Qiaexll, Qiagen, Hilden). Ca. 40 ng der DNA wurden in 50 ul Ligationspuffer mit 1 u T4-DNA-Ligase für 20 h bei 16°C inkubiert. Anschließend wurde der Ligationsansatz durch Mikrodialyse entsalzt und 5 µl zur Transformation von XL1-Blue Zellen durch Elektroporation eingesetzt. Die transformierten Zellen wurden auf dYT Agar mit 100 µg/ml Ampicillin ausplattiert.

### 6.) Zweite Bibliotheksanalyse

Die Transfonnanten sollten die gewünschte Bibliothek an Codon-mutagenisierten Varianten von GFP enthalten. Zur Analyse dieser Bibliothek wurden daher 5 Transformanten zufällig ausgewählt und die gesamte Sequenz von GFP bestimmt, um Ort und Art der Mutation festzustellen. Folgende Mutationen wurden gefunden: S86W (AGT->TGG), G51H (GGA->CAC), N164R (AAC->CGC) und V219N (GTC->AAG). Eine Mutation war nicht im richtigen Leserahmen CAATCT->CTCGCT und führte zur Doppelmutation Q204L, S205A.

### 6.) Phänotypische Analyse

Die Bibliothek an Varianten von GFP kann an dieser Stelle auf Varianten untersucht werden, die eine gewünschte phänotypische Veränderungen (z.B. erhöhte Expression von GFP, Verschiebung von Anregungs- und/oder Emissionswellenlänge, etc.) zeigen. Gewünschte Varianten können dann isoliert und ihren Eigenschaften gerecht eingesetzt werden.

### Literaturverzeichnis

1. Rubingh, D.N., Protein engineering from a bioindustrial point of view. *Curr. Opin. Biotechnol.,* 1997. **8**(4): p. 417-22.
2. Chen, R., Enzyme engineering: rational redesign versus directed evolution. *Trends Biotechnol.,* 2001. **19**(1): p. 13-14.
3. Petrounia, I.P. and F.H. Arnold, Designed evolution of enzymes. *Curr. Opin. Biotechnol.,* 2000. **11**: p. 325-330.
4. Dordick, J.S., Y.L. Khmelnitsky, and M.V. Sergeeva, The evolution of biotransformation technologies. *Curr. Opin. Microbiol.,* 1998. **1**: p. 311-318.
5. Stemmer, W.P., DNA shuffling by random fragmentation and reassembly: in vitro recombination for molecular evolution. *Proc. Natl. Acad. Sci. USA,* 1994. **91**(22): p. 10747-51.
6. Volkov, A.A., Z. Shao, and F.H. Arnold, Recombination and chimeragenesis by in vitro heteroduplex formation and in vivo repair. *Nucleic Acids Res.,* 1999. **27**(18): p. e18.
7. Zhao, H., et al., Molecular evolution by staggered extension process (StEP) in vitro recombination. *Nat. Biotechnol.,* 1998. **16**(3): p. 258-61.
8. Fromant, M., S. Blanquet, and P. Plateau, Direct random mutagenesis of gene-sized DNA fragments using polymerase chain reaction. *Anal. Biochem.,* 1995. **224**(1): p. 347-53.
9. Lahr, S.J., et al., Pattemed library analysis: A method for the quantitative assessment of hypotheses concerning the determinants of protein structure. *Proc. Nat. Acad Sci. USA,* 1999. **96**(26): p. 14860-14865.
10. Greener, A., M. Callahan, and B. Jerpseth, An efficient random mutagenesis technique using an *E. coli* mutator strain. *Mol. Biotechnol.,* 1997. **7**(2): p. 189-95.
11. Crameri, A., et al., DNA shuffling of a family of genes from diverse species accelerates directed evolution. *Nature,* 1998. **391**(6664)**:** p. 288-91.
12. Tindall, K.R. and T.A. Kunkel, Fidelity of DNA synthesis by the Thermus aquaticus DNA polymerase. *Biochemistry,* 1988. **27**(16): p. 6008-13.
13. Spee, J.H., W.M. de Vos, and O.P. Kuipers, Efficient random mutagenesis method with adjustable mutation frequency by use of PCR and dITP. *Nucleic Acids Res.,* 1993. **21**(3): p. 777-8.
14. Sawano, A. and A. Miyawaki, Directed evolution of green fluorescent protein by a new versatile PCR strategy for site-directed and semi-random mutagenesis. *Nucleic Acids Res.,* 2000. **28**(16): p. e78.
15. May, O., P.T. Nguyen, and F.H. Arnold, Inverting enantioselectivity by directed evolution of hydantoinase for improved production of L-methionine. *Nat. Biotechnol.,* 2000. **18**(3): p. 317-20.
16. Kuchner, O. and F.H. Arnold, Directed evolution of enzyme catalysts. *Trends Biotechnol.,* 1997. **15**(12): p. 523-30.
17. Cadwell, RC. and G.F. Joyce, Mutagenic PCR. *PCR Methods Appl.,* 1994. **3**: p. S136-S139.
18. Komar, A.A., T. Lesnik, and C. Reiss, Synonymous codon substitutions affect ribosome traffic and protein folding during in vitro translation. *FEBSLett.,* 1999. **462**(3): p. 387-91.
19. Martin, A.B. and P.G. Schultz, Opportunities at the interface of chemistry and biology. *Trends Cell. Biol.,* 1999*.* **9**(12): p. M24-8.
20. Oliphant, A.R., A.L. Nussbaum, and K. Struhl, Cloning of random-sequence oligodeoxynucleotides. *Gene,* 1986*.* **44**(2-3): p. 177-83.
21. Balint, R.F. and J.W. Larrick, Antibody engineering by parsimonious mutagenesis. *Gene,* 1993. **137**(1): p. 109-18.
22. Tomandl, D., A. Schober, and A. Schwienhorst, Optimizing doped libraries by using genetic algorithms. *J. Comput. Aided Mol. Des.,* 1997. **11**(1): p. 29-38.
23. Gaytan, P., et al., Combination of DMT-mononucleotide and Fmoc-trinucleotide phosphoramidites in oligonucleotide synthesis affords an automatable codon-level mutagenesis method. *Chem. Biol.,* 1998. **5**(9): p. 519-27.
24. Kayushin, A.L., et al., A convenient approach to the synthesis of trinucleotide phosphoramidites--synthons for the generation of oligonucleotide/peptide libraries. *Nucleic Acids Res.,* 1996. **24**(19): p. 3748-55.
25. Lyttle, M.H., et al., Mutagenesis using trinucleotide beta-cyanoethyl phosphoramidites. *Biotechniques,* 1995. 19(2): p. 274-81.
26. Ono, A., et al., The synthesis of blocked triplet-phosphoramidites and their use in mutagenesis. *Nucleic Acids Res.,* 1995. **23**(22): p. 4677-82.
27. Virnekas, B., et al., Trinucleotide phosphoramidites: ideal reagents for the synthesis of mixed oligonucleotides for random mutagenesis. *Nucleic Acids Res.,* 1994. **22**(25): p. 5600-7.
28. Smith, A.D., *Oxford Dictionary of Biochemistry and Molecular Biology.* 2 ed. 2000, Oxford: University Press.
29. McDonald, C.J., *Enzymes in Molecular Biology.* Essential Data Series, ed. D. Rickwood and B.H. Hames. 1996, Chichester. John Wiley & Sons.
30. U.S. Pat. No. 4 683 195
31. U.S. Pat. No. 4 683 202
32. Hennecke, J., P. Sebbel, and R Glockshuber, Random circular pennutaüon of DsbA reveals segments that are essential for protein folding and stability. *J. Mol. Biol.,* 1999. **286**(4): p. 1197-215.
33. Stone, J.C., et al., Identification of functional regions in the transforming protein of Fujinami sarcoma virus by in-phase insertionmutagenesis. *Cell,* 1984. **37**(2): p. 549-58.
34. Horton, RM. and L.R Pease, *Recombination and mutagenesis of DNA sequences using PCR,* in *Directed Mutagenesis - A Pradical Approach,* M.J. McPherson, Editor. 1991, IRL Press: Oxford. p. 217-247.
35. Eisinger, D.P., et al., Long-inverse PCR to generate regional peptide libraries by codon mutagenesis. *Biotechniques,* 1997. **22**(2): p. 250-254.
36. Hayes, F., et al., Pentapeptide scanning mutagenesis: encouraging old proteins to execute unusual tricks. *Trends in Microbiology,* 2000. **8**(12): p. 571-577.
37. Kegler-Ebo, D., et al., Codon cassette mutagenesis: a general method to insert or replace individual codons by using universal mutagenic cassettes. *Nucleic Acid Res.,* 1994. **22**(9): p. 1593-1599.
38. Chen, W.-J., et al., A modified Kanamycin-resistance cassette to facilitate two-codon insertion mutagenesis. *Gene,* 1992. **111**(1): p. 143-144.

## Patentansprüche

1. Methode zur Herstellung von Sequenzvariation in DNA, **gekennzeichnet durch** die Teilschritte:
a) Einführung eines Transposons (DONOR) in besagte DNA (GEN) an verschiedenen, zufälligen Positionen und
b) gezieltes Entfernen von DONOR aus GEN und gezieltes Entfernen einer definierten Anzahl nebeneinander liegender Nukleotide von GEN aus GEN, derart, dass an exakt der Position dieser entfernten Nukleotide innerhalb der Sequenzabfolge von GEN eine definierte Anzahl von Nukleotiden, die ursprünglich aus DONOR stammen und die vollständig degeneriert sein können, in GEN verbleiben.

2. Methode nach Patentanspruch 1, **dadurch gekennzeichnet, dass** Teilschritt 1 (b) durch mehrere Zyklen von Teilschritten (a) bis (d) erfolgt, gefolgt von den Teilschritten (e) bis (g):
a) Restriktion der GEN und zumindest Teile von DONOR enthaltenden DNA mit mindestens einem Restriktionsenzym des Typs IIs,
b) bei Bedarf Behandlung der DNA-Enden mit Enzymen, die die DNA-Enden glätten und/oder Isolation des GEN enthaltenden Teils der restringierten DNA,
c) intramolekulare Ligation der freien DNA-Enden des GEN enthaltenden Teils der restringierten DNA unter Bildung eines zirkulären DNA-Strangs der durch diese Ligation eine neue Erkennungsstelle für ein Restriktionsenzym des Typs IIs erhält und
d) bei Bedarf Isolation und/oder Amplifikation dieses zirkulären DNA-Strangs.
e) Restriktion der aus dem letzten Zyklus in Teilschritt (c) erhaltenen und bei Bedarf in dem letzten Zyklus in Teilschritt (d) amplifizierten und/oder isolierten Produkte mit mindestens einem Restriktionsenzym des Typs Ils,
f) Behandlung der DNA-Enden mit Enzymen, die die DNA-Enden glätten und/oder Isolation des GEN enthaltenden Teils der restringierten DNA
g) intramolekulare Ligation der freien DNA-Enden des GEN enthaltenden Teils der restringierten DNA unter Bildung eines zirkulären DNA Strangs (cGEN'), der die ursprüngliche Sequenzabfolge von GEN aufweist, mit Ausnahme von wenigen Nukleotiden aus GEN die durch degenerierte Nukleotide aus DONOR ersetzt sind.

3. Methode nach Patentanspruch 1, **dadurch gekennzeichnet, dass** nach Teilschritt 1 (a) eine Amplifikation der Produkte von Teilschritt 1 (a) erfolgt.

4. Methode zur Herstellung von Sequenzvariation in DNA, **gekennzeichnet durch** die Teilschritte:
a) Einführung eines Doppelstrangbruchs in besagte DNA (GEN),
b) Ligation eines DNA Strangs (DONOR) zu beiden **durch** den Doppelstrangbruch in GEN gebildeten DNA-Enden von GEN unter Bildung eines Ligationsproduktes (LP),
c) Herausschneiden des überwiegenden Teils von DONOR aus LP mit Ausnahme von wenigen Nukleotiden die degeneriert sein können und Herausschneiden eines kleinen Teils von GEN aus LP,
d) intramolekulare Ligation der freien DNA-Enden des verbleibenden Teils von LP unter Bildung eines zirkulären DNA Strangs (cGEN'), der die ursprüngliche Sequenzabfolge von GEN aufweist, mit Ausnahme von wenigen Nukleotiden aus GEN die **durch** degenerierte Nukleotide aus DONOR ersetzt sind.

5. Methode nach Patentanspruch 4, **dadurch gekennzeichnet, dass** nach Teilschritt 4 (b) eine Amplifikation und/oder Isolation der Produkte der Ligation aus DONOR mit dem gespaltenen GEN (LP) erfolgt.

6. Methode nach Patentanspruch 4, **dadurch gekennzeichnet, dass** das Produkt von Teilschritt 4 (d), cGEN' für eine neue Runde der Sequenzvariation als GEN' verwendet wird.

7. Methode nach Patentansprüchen 1 und 4, **dadurch gekennzeichnet, dass** DONOR eine definierte Zahl degenerierter Nukleotide enthält.

8. Eine Methode nach Patentanspruch 4, **gekennzeichnet dadurch, dass** bei 2 (b) jeweils beide Enden von DONOR zu jeweils beiden Enden von GEN ligiert werden unter Bildung eines zirkulären DNA Strangs.

9. Eine Methode nach Patentansprüchen 1 - 5, **gekennzeichnet dadurch, dass** DONOR ein in Mikroorganismen selektierbares Gen enthält.

10. Methode nach Patentanspruch 4, **dadurch gekennzeichnet, dass** bei 4 (b) jeweils ein DNA-Ende von zwei verschiedenen Molekülen von DONOR zu den beiden DNA-Enden von einem Molekül GEN ligiert werden.

11. Eine Methode nach Patentanspruch 4, **gekennzeichnet dadurch, dass** Ligationsprodukte zwischen DONOR und GEN über spezifische Bindungseigenschaften z.B. an magnetische Kügelchen von unligierten Molekülen GEN getrennt werden.

12. Methode nach Patentanspruch 4, **dadurch gekennzeichnet, dass** bei 4 (c)
a) der überwiegende Teil von DONOR und ein kleiner Teil von GEN durch die Reaktion mindestens eines Restriktionsenzyms des Typs IIs von LP abgespalten wird und
b) die entstandenen DNA-Enden durch die Behandlung mit mindestens einer Nuklease und/oder Polymerase geglättet werden.

13. Methode nach Patentansprüchen 1, 2, 4 und 12, **dadurch gekennzeichnet, dass** DONOR bis auf 2, 3, 4, 5, oder 6 Nukleotide komplett von GEN abgespalten wird und dass der vom Hauptteil von GEN abzuspaltende kleine Teil von GEN 2, 3, 4, 5, oder 6 Nukleotide lang ist.

14. Methode nach Patentansprüchen 1, 2, 4 und 12, **dadurch gekennzeichnet, dass** DONOR von GEN komplett bis auf genau 3 Nukleotide abgespalten wird und dass genau 3 Nukleotide, die aus GEN stammen von GEN abgespalten werden.

## Claims

1. A method for producing sequence variation in DNA, which comprises the steps of:
a) Incorporation of a transposon (DONOR) into said DNA (GEN) at different random positions and
b) specific removal of DONOR from GEN and specific removal of a defined number of adjacent nucleotides of GEN from GEN, such that at exactly the position of these removed nucleotides within the sequence of GEN a defined number of nucleotides remain that originate from DONOR and which can be completely degenerate.

2. A method according to claim 1, wherein step 1 (b) occurs by several cycles of the following steps (a) to (d), followed by the steps (e) to (g):
a) restriction digestion of GEN and at least parts of DONOR containing DNA using a restriction endonuclease of type IIs
b) by demand, treatment of the DNA-ends with enzymes that make the DNA-ends blunt and/or isolation of the GEN containing part of the restricted DNA
c) intramolecular ligation of the free DNA ends of the GEN containing part of the restricted DNA by which a circular strand of DNA is formed, which such receives a new recognition site for a restriction enzyme of type Ils and
d) by demand isolation and/or amplification of this circular DNA-strand.
e) restriction digestion using at least one restriction enzyme of type Ils of the products obtained from the last cycle in step (c) or, when necessary of the amplified and/or isolated products from the last cycle in step (d)
f) treatment of the DNA-ends with enzymes that make DNA-ends blunt and/or isolation of the GEN containing part of the restricted DNA
g) intramolecular ligation of the free DNA-ends of the GEN containing part of the restricted DNA by which a circular DNA strand (cGEN') is formed, which has the original sequence of GEN with the exception of few nucleotides of GEN that were replaced by degenerate nucleotides from DONOR.

3. A method according to claim 1, wherein, after step 1 (a) the products of step 1 (a) are amplified.

4. A method for producing sequence variation in DNA, comprising the steps:
a) Introduction of a double strand breakage in said DNA (GEN),
b) Ligation of a DNA Strand (DONOR) to both DNA-ends of GEN, formed by the double strand breakage of GEN, producing a ligation product (LP),
c) Removal of the major part of DONOR from LP apart from few nucleotides that can be degenerate and removal of a small part of GEN from LP
d) intramolecular ligation of the free DNA-ends of the remaining part of LP such that a circular DNA strand (cGEN') is formed, which has the original sequence of GEN with the exception of few nucleotides of GEN that were replaced by degenerate nucleotides from DONOR.

5. A method according to claim 4, wherein, after step 4 (b) an amplification and/or isolation is performed of the products of the ligation (LP) between DONOR and the broken GEN.

6. A method according to claim 4, wherein, the product of step 4 (d), cGEN' is used as GEN' for an additional round of sequence variation.

7. A method according to claims 1 and 4, wherein DONOR contains a defined number of degenerate nucleotides.

8. A method according to claim 4, wherein at step 4 (b) each of both ends of DONOR is ligated to each of both ends of GEN such forming a circular strand of DNA.

9. A method according to claims 1-5, wherein DONOR contains a gene that can be selected for in microorganisms.

10. A method according to claim 4, wherein at step 4 (b) one DNA-end each of two different molecules of DONOR is ligated with both DNA-ends of one molecule GEN.

11. A method according to claim 4, wherein ligation products between DONOR and GEN are separated from unligated molecules of GEN by using specific binding properties, e.g. via binding to magnetic beads.

12. A method according to claim 4, wherein at step 4 (c)
a) the major part of DONOR and a small part of GEN are cleft from LP by the action of at least on restriction enzyme of type Ils and
b) the created DNA-ends are made blunt by treatment with at least one nuclease and/or polymerase.

13. A method according to claims 1, 2, 4 and 12, wherein DONOR is completely cleft from GEN, apart from 2, 3, 4, 5, or 6 nucleotides and wherein the smaller part of GEN that is to be cleft from the major part of GEN has a length of 2, 3, 4, 5, or 6 nucleotides.

14. A method according to claims 1, 2, 4 and 12, wherein DONOR is completely cleft from GEN, apart from exactly 3 nucleotides and wherein exactly 3 nucleotides, originating from GEN are split off GEN.

## Revendications

1. Procédé pour produire la variation de la séquence de l'ADN, **caractérisé en ce que** il comprend les étapes:
a) L'incorporation d'un transposon (DONATEUR) dans ladite ADN (GEN) à différentes positions aléatoires et
b) à déplacement spécifique de DONATEUR de GEN et à déplacement spécifique d'un nombre défini de nucléotides adjacents de GEN de GEN, tels qu'exactement à la position de ces nucléotides éliminés dans la séquence de la GEN un nombre défini de nucléotides demeurent qui proviennent du DONATEUR et qui peut être complètement dégénérée.

2. Procédé selon la revendication 1, **caractérisé en ce que** étape 1 (b) se produit par plusieurs cycles des étapes suivantes (a) à (d), a suivi des étapes (e) à (g):
a) digestion de restriction de la GEN et au moins des parties du DONATEUR contenant l'ADN en utilisant une endonucléase de restriction de type Ils
b) par la demande, traitement des ADN extrémités avec des enzymes qui rendent les ADN extrémités franche et/ou l'isolement de la GEN contenant une partie de ADN restreinte
c) ligature intramoléculaire des fins libres de ADN de la GEN contenant une partie de ADN restreinte par laquelle une molécule circulaire de ADN est formée, que telle reçoit un nouvel emplacement d'identification pour une enzyme de restriction de type IIs et
d) par l'isolement de demande et/ou l'amplification de cette ADN molécule circulaire.
e) digestion de restriction en utilisant au moins une enzyme de restriction de type IIs des produits obtenus à partir du dernier cycle dans l'étape (c) ou, si nécessaire des produits amplifiés et/ou d'isolement du dernier cycle dans l'étape (d)
f) traitement des ADN extrémités avec des enzymes qui rendent des ADN extrémités franche et/ou l'isolement de la GEN contenant une partie de l'ADN restreinte
g) ligature intramoléculaire des ADN extrémités libres de la GEN contenant une partie de l'ADN restreinte par laquelle une molécule circulaire d'ADN (cGEN') est formée, qui a la séquence original de la GEN excepté peu de nucléotides de GEN qui ont été remplacés par les nucléotides dégénérés du DONATEUR.

3. Procédé selon la revendication 1, **caractérise en ce que** après l'étape 1 (a) les produits de l'étape 1 (a) sont amplifiés.

4. Procédé pour produire la variation de la séquence de l'ADN, **caractérisé par** les étapes:
a) Introduction d'une double rupture de molécule en ladite ADN (GEN),
b) ligature d'une molécule d'ADN (DONATEUR) aux deux ADN extrémités de GEN, constituées par la double rupture de molécule de la GEN, produisant un produit de ligature (LP),
c) déplacement de la majeure partie du DONATEUR de LP à l'exception de peu de nucléotides qui peuvent être dégénérés et déplacement d'une petite partie de GEN de LP d) ligature intramoléculaire des ADN extrémités libres de la partie restante du LP tels qu'une molécule circulaire d'ADN (cGEN') est formée, qui a l'ordre original de la GEN excepté peu de nucléotides de GEN qui ont été remplacés par les nucléotides dégénérés du DONATEUR.

5. Procédé selon la revendication 4, **caractérisé en ce que** après qu'étape 4 (b) un amplification et/ou isolement soit exécutée des produits de la ligature (LP) entre le DONATEUR et la GEN cassée.

6. Procédé selon la revendication 4, **caractérisé en ce que**, le produit de l'étape 4 (d), cGEN' est employé comme GEN' pour un rond additionnel de variation de la séquence.

7. Procédé selon les revendications 1 et 4, **caractérisé en ce que** le DONATEUR contient un nombre défini de nucléotides dégénérés.

8. Procédé selon la revendication 4, **caractérisé en ce que** à l'étape 4 (b) chacun des deux extrémités de DONATEUR est ligaturé à chacune des deux extrémités de GEN une telle formation d'une molécule circulaire de l'ADN.

9. Procédé selon les revendications 1 - 5, **caractérisé en ce que** le DONATEUR contient un gène qui peut être sélecté pour dans des micro-organismes.

10. Procédé selon la revendication 4, **caractérisé en ce que** à l'étape 4 (b) une ADN extrémité chacune de deux molécules différentes de DONATEUR est ligaturée avec les deux ADN extrémités d'une molécule GEN.

11. Procédé selon la revendication 4, **caractérisé en ce que** des produits de ligature le DONATEUR et la GEN sont séparés de des molécules de GEN non ligaturé au DONATEUR en employant les propriétés de liage spécifiques, par exemple par l'intermédiaire de lier aux perles magnétiques.

12. Procédé selon la revendication 4, **caractérisé en ce que** à l'étape 4 (c)
a) la majeure partie du DONATEUR et une petite partie de GEN sont séparé du LP par l'action au moins de une enzyme de restriction du type Ils et
b) les ADN extrémités créées sont rendues franche par traitement avec au moins une nucléase et/ou polymérase.

13. Procédé selon les revendications 1, 2, 4 et 12, **caractérisé en ce que** le DONATEUR est complètement fendu de la GEN, à l'exception de 2, 3, 4, 5, ou 6 nucléotides et où la partie plus petite de GEN qui doit pour être fendus de la majeure partie de GEN a une longueur de 2, 3, 4, 5, ou 6 nucléotides.

14. Procédé selon les revendications 1, 2, 4 et 12, **caractérisé en ce que** le DONATEUR est complètement fendu de la GEN, à l'exception d'exactement 3 nucléotides et où exactement 3 nucléotides, provenant de la GEN, sont éliminés de la GEN.
